# EUROPEAN PATENT SPECIFICATION

(11) **EP 2 985 049 B1**
(45) Date of publication and mention of the grant of the patent: **12.06.2019**
(21) Application number: 14782864.4
(22) Date of filing: 03.04.2014
(51) Int. Cl.: A61M 5/32, A61M 5/158, A61M 25/06

(54) **BUTTERFLY-TYPE NEEDLE ASSEMBLY AND BUTTERFLY-TYPE NEEDLE PROTECTIVE SLEEVE**
SCHMETTERLINGSNADELANORDNUNG UND SCHMETTERLINGSNADELSCHUTZHÜLSE
ENSEMBLE AIGUILLE À AILETTES ET MANCHON DE PROTECTION D'AIGUILLE À AILETTES

(30) Priority: 10.04.2013 CN 201310122433
(43) Date of publication of application: 17.02.2016
(73) Proprietor: Wellspring Medical Supplies Co., Ltd., Shanghai 200131 (CN)
(72) Inventor: QU, Wanqing, Shanghai 200131 (CN)
(74) Representative: Capasso, Olga
(86) International application number: PCT/CN2014/074716
(87) International publication number: WO 2014/166353

(56) References cited:
- WO-A1-2010/038844
- WO-A1-2012/032378
- CN-A- 1 520 892
- CN-A- 101 443 059
- CN-A- 102 325 557
- CN-A- 103 191 496
- CN-U- 202 314 697
- CN-U- 203 208 454
- CN-Y- 2 181 300
- JP-U- H0 670 744
- US-A- 5 120 320
- US-A1- 2004 153 039
- US-A1- 2006 100 585

## Description

### DESCRIPTION

### TECHNICAL FIELD

The present invention relates to a medical device and, and particularly to a needle protective sleeve for use in clinical puncture infusion/blood collection.

### BACKGROUND ART

At present, there are numerous structures designed for medical butterfly needles; according to searches, patent documents WO 2012032378 A1, WO 2010038844 A1, CN 03102395.9, CN 201110308424.0, CN 201080008965.5 all relate to the solutions for protection of butterfly needles, among which, WO 2010038844 A1 discloses an anti-puncture system, in which a housing assembly has a main body component, the main body component has a far end, a near end and a barrel between the far end and the near end, the barrel is the main lengthwise portion of the main body component, the near end and the far end are respectively formed with a hole, the main body part is composed of an upper cap and a lower cap, the upper cap and the lower cap are connected through a pair of flexible hinges, the upper end of the flexible hinge is connected to the far end of the upper cap and the lower end is connected to the far end of the lower cap, such that the upper cap and the lower cap can be operated between a closed and an open position. The butterfly needle having been used and contaminated can retract back into the main body part, and can be clamped by a slot formed between the opposite edges of the upper cap and the lower cap, such that the butterfly needle is located in a barrel of a safety needle sleeve, so as to achieve the purpose of safely recovering the butterfly needle.

In practice, the current butterfly-type needle protective sleeves are more or less flawed, for example, the clamping of the contaminated needle is not firm, leaving the needle exposed outside the needle sleeve, thereby causing the risk of personnel suffering from needle prick and infection by virus.

Pertinent devices are described in US 2004/153039 A1.

### SUMMARY OF THE INVENTION

An object of the present invention is to provide a butterfly-type needle assembly and a butterfly-type needle protective sleeve, which can provide better protection for a user.

The invention is defined by the subject-matter of claim 1.

The butterfly-type needle protective sleeve provided for achieving said object comprises a body for placing a butterfly-type needle therein, said body being made of a plastic material, said butterfly-type needle comprising a needle portion, a wing portion protruding out of said body, and a handle portion fixed onto said wing portion; one end of said handle portion being connected to said needle portion and the other end being used for connecting to a pipe, and said butterfly-type needle, with respect to said protective sleeve, having a using state in which said needle portion protrudes and a retracted state in which said needle portion retracts, wherein said body comprises a needle tip protective head, an upper cap and a lower cap; said needle tip protective head is a cylinder with an axially penetrating needle hole; said upper cap and said lower cap are elongated components extending in an axial direction of said body and respectively have a far end and a near end; said far ends of said upper cap and said lower cap are connected to said cylinder through flexible hinges; said near end of said upper cap is detachably connected with said near end of said lower cap in a clamping manner; said upper cap and said lower cap can be allowed to move between an open state and a closed state by the aid of said flexible hinges; a channel for allowing said handle portion to move axially is arranged between said upper cap and said lower cap in the closed state; a clamping structure is formed between said upper cap and said lower cap and can match with said wing portion of said butterfly-type needle to enable said butterfly-type needle to be clamped in the using state or be locked in the retracted state; said butterfly-type needle can be allowed to move axially to the retracted state under a designed outer force by the aid of said clamping structure; said needle hole of said needle tip protective head is used for accommodating the axial movement of said needle portion; the needle tip of said needle portion in the retracted state is accommodated in the needle tip protective head, and a circumferential side corresponding to the needle tip is of a fully enclosed structure.

In the butterfly-type needle protective sleeve, said body is integrally injection-moulded.

In an embodiment of the butterfly-type needle protective sleeve, an upper edge of said lower cap adjacent to said far end is formed as a plane. In the butterfly-type needle protective sleeve according to the invention, a lower edge of said upper cap adjacent to said far end is formed with a continuous guide slope, so as to guide said wing portion when pulling said butterfly-type needle in the using state towards the near end, so that said butterfly-type needle smoothly moves axially into said retracted state.

In an embodiment of the butterfly-type needle protective sleeve, the lower edge in the middle part of said upper cap protrudes downward until the upper edge of said lower cap, such that in said closed state, the middle parts of said upper cap and said lower cap are of fully enclosed structures.

In an embodiment of the butterfly-type needle protective sleeve, the lower edge of said upper cap adjacent to said downward protrusion is formed with a recessed portion for receiving said wing portion, and the recessed portion is provided with a side wall for matching with said wing portion in a 90 degree clamped manner.

In an embodiment of the butterfly-type needle protective sleeve, said lower cap is formed with a first upward protrusion on the inner side of said downward protrusion, and said first upward protrusion is used for matching with said wing portion in a 90 degree clamped manner.

In an embodiment of the butterfly-type needle protective sleeve, the lower edge of said lower cap below said recessed portion is formed with a second upward protrusion, and said second upward protrusion is used for clamping with an orifice slot of said wing portion.

The butterfly-type needle assembly provided for achieving said object comprise a butterfly-type needle and a butterfly-type needle protective sleeve of any one of the embodiments mentioned above.

In an embodiment of the butterfly-type needle assembly, said wing portion has a front face and a back face, said front face and the lower side of said upper cap are opposite to each other, said back face and the upper side of said lower cap are opposite to each other, a blade face groove of the needle tip of said needle portion and said front face are oriented in the same direction, and said needle handle is located on the back face side of said wing portion.

In an embodiment of the present invention, since the protective sleeve has a needle tip protective head, after the needle tip retracts, the periphery thereof is completely enclosed, and therefore it will not cause accidental puncture injury, or the risk of infection caused by puncture. Moreover, since the protective head is an integrally moulded part, the structure is simple, the surface is rounded or smooth, there is no projection, having a better feel, so that there is a better protection for the user.

### DESCRIPTION OF THE DRAWINGS

The particular features and properties of the present invention will be further given by the following embodiments and the accompany figures.
Fig. 1 is a perspective view of a butterfly-type needle assembly (viewed from the front).
Fig. 2 is a perspective view of the butterfly-type needle assembly (viewed from the back).
Fig. 3 is a side view of the butterfly-type needle assembly.
Fig. 4 is a perspective view of a butterfly-type needle protective sleeve in an open state.
Fig. 5 is a front view of the butterfly-type needle protective sleeve.
Fig. 6 is a side view of the butterfly-type needle protective sleeve.
Fig. 7 is a perspective view of the butterfly-type needle protective sleeve in a closed state.
Fig. 8 is a perspective view of the butterfly-type needle assembly in a retracted state.
Fig. 9 is a front view of the butterfly-type needle assembly in the retracted state, with an upper cap being omitted.
Fig. 10 is a side view of the butterfly-type needle assembly in the retracted state, with the upper cap being omitted.
Fig. 11 is a front view of a butterfly-type needle.
Fig. 12 is a left view of the butterfly-type needle.
Fig. 13 is a top view of the butterfly-type needle.
Fig. 14 is a perspective view of the butterfly-type needle.
Fig. 15 is a schematic view of a clinical puncture infusion/blood collection system.

### PREFERRED EMBODIMENTS OF THE INVENTION

The present invention will be further described below in conjunction with particular embodiments and the accompanying drawings, and more details are explained in the following description, in order to fully understand the present invention; however, the present invention can obviously be implemented in manners different than that described herein, a person skilled in the art can make similar extensions and deductions without departing from the connotation of the invention according to the practical applications, and therefore the scope of protection of the present invention should not be limited to the contents of the particular embodiments herein.

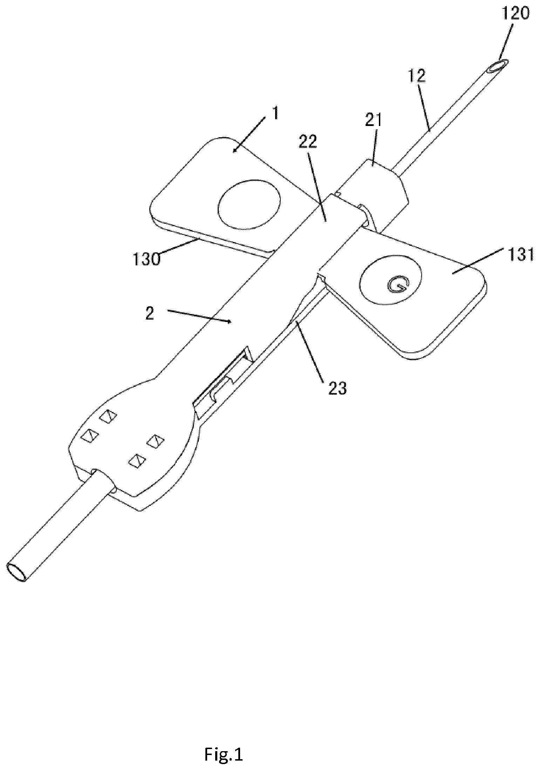

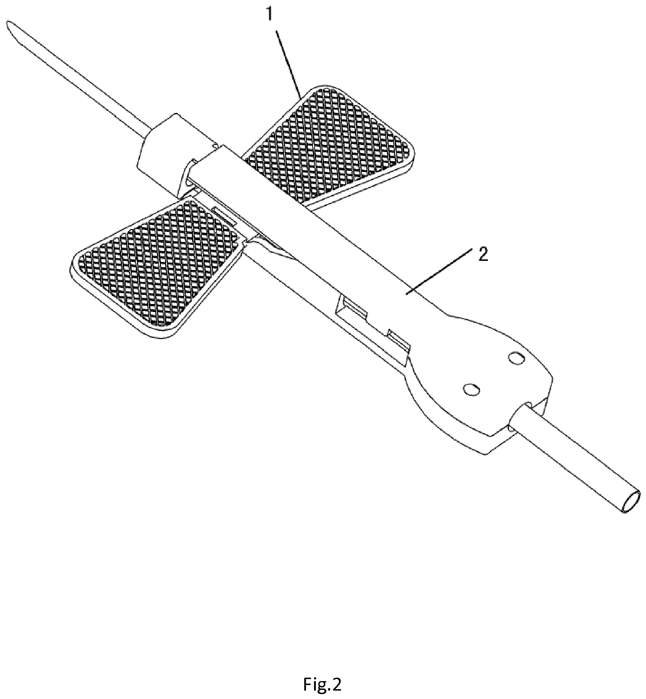

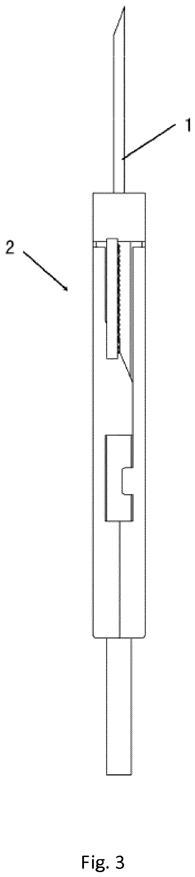

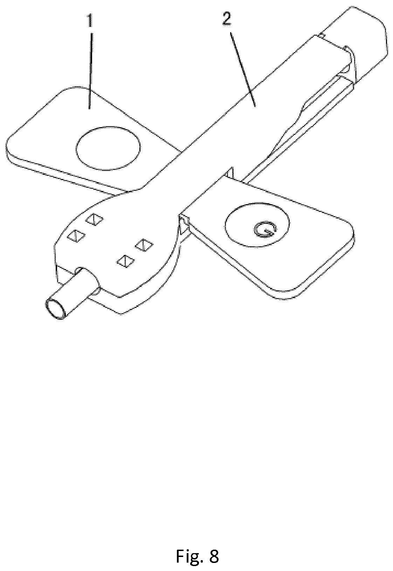

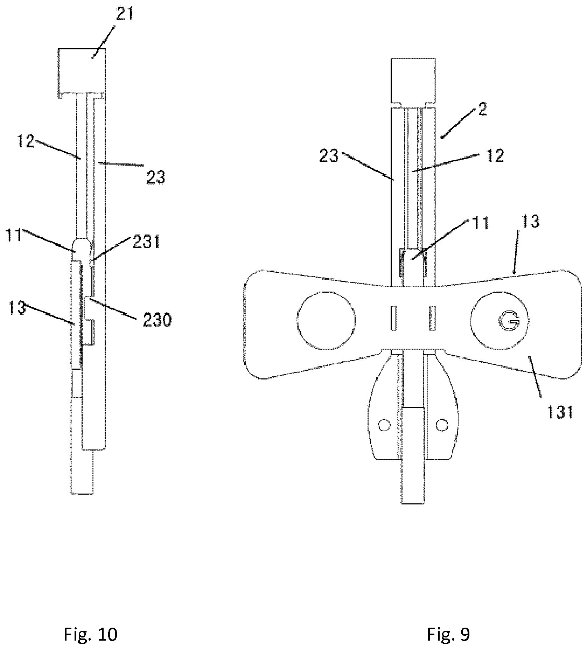

As shown in Figs. 1-3, the butterfly-type needle assembly comprises a butterfly-type needle 1 and a protective sleeve 2. The butterfly-type needle 1 is housed or accommodated or received by the protective sleeve 2, as in the state shown in Figs. 1-3, a needle portion of the butterfly-type needle 1 pass out of the protective sleeve 2 and is in an unprotected using state, at which time the user can use the needle portion for puncturing with the aid of a wing portion. As shown in Figs. 8-10, the needle portion of the butterfly-type needle 1 retracts into the protective sleeve 2, and is in a protected retracted state.

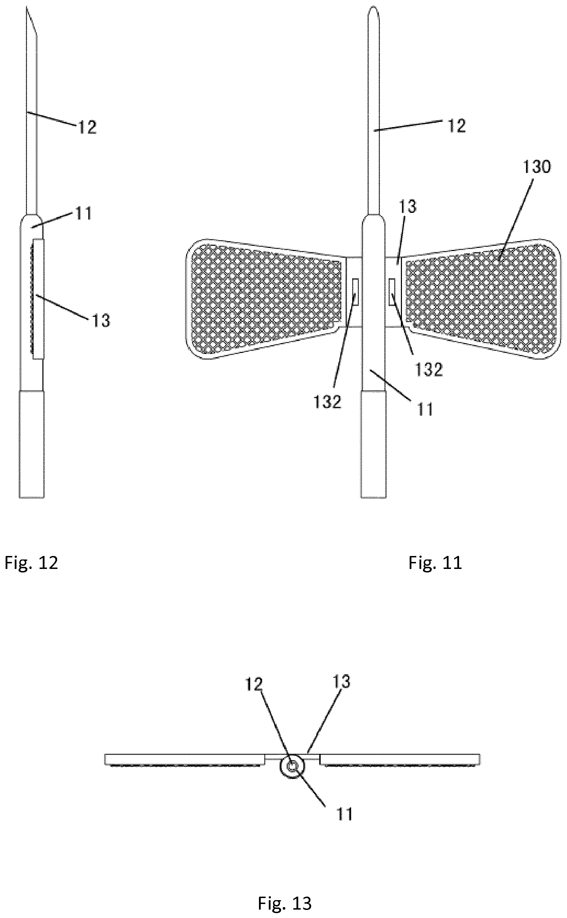

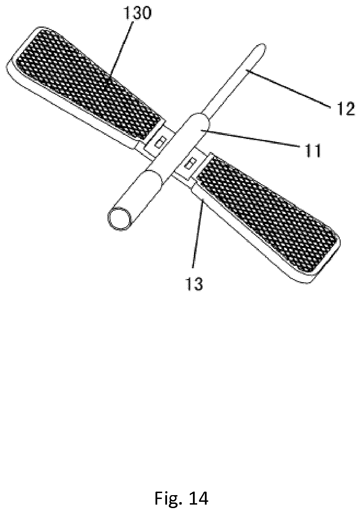

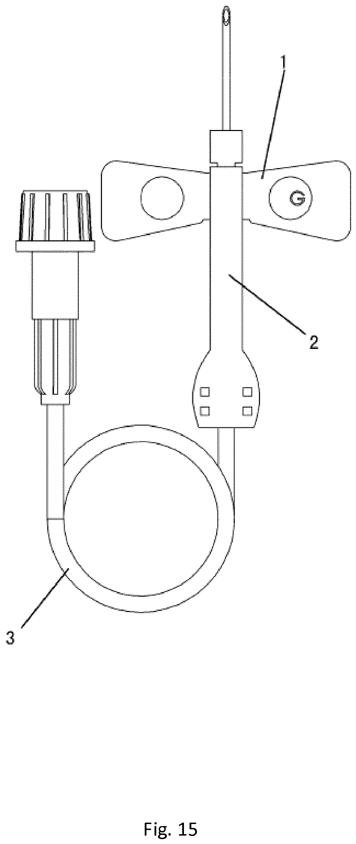

As shown in Figs. 11-14, the butterfly-type needle 1 has the needle portion 12, a handle portion 11 and the wing portion 13, the wing portion 13 having a front face 131 and a back face 130, the back face 130 is typically provided with an anti-slip surface, and the front face 131 is typically provided with a label and is usually a smooth surface. The handle portion 11 is fixed to the middle part of the back face 130 of the wing portion 13, and the front end of the handle portion 11 is connected to the needle portion 12 while the rear end is generally connected to a hose 3 (as shown in Fig. 15).

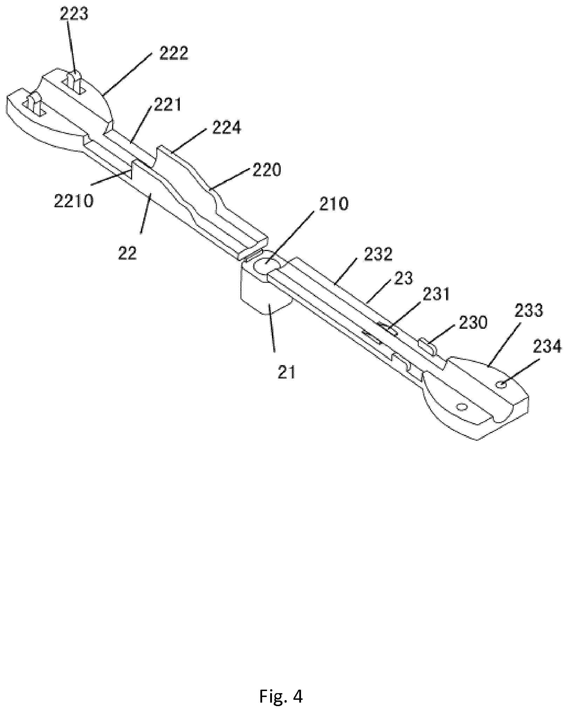

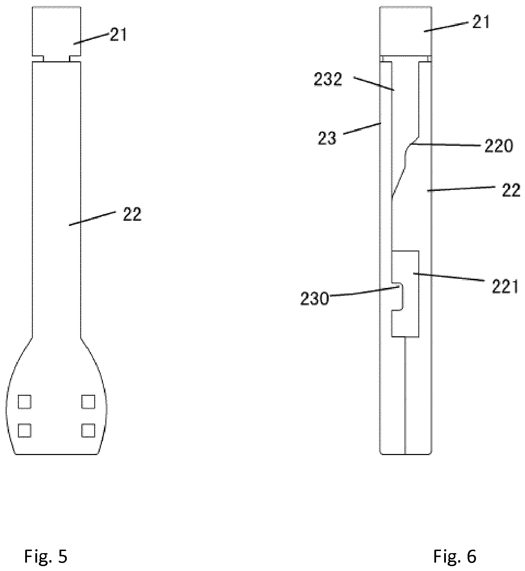

As shown in Fig. 4-7, the protective sleeve 2 is an integrally injection-moulded part, and comprises a needle tip protective head 21, an upper cap 22 and a lower cap 23, and the upper cap 22 and the lower cap 23 are elongated components extending in an axial direction of the protective sleeve 2 and respectively have a far end and a near end. With respect to the orientation terms described above and hereafter, for example, upper, lower, far, near, front, back and the like, reference is generally made to the state in which the butterfly-type assembly is normally used by the user. The needle tip protective head 21 a cylinder with an axially penetrating needle hole 210 in the centre, which is used for receiving the needle tip of the needle portion 12 in the retracted state, and an enclosed structure is provided in the circumferential direction around the needle tip, in order to play the role of protecting the personnel, avoiding the needle tip from pricking the personnel.

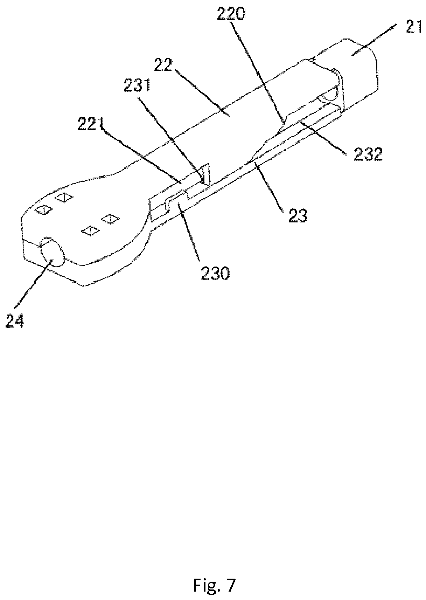

The upper cap 22 and the lower cap 23 are respectively connected to the upper and lower sides of one end of the needle tip protective head 21 by flexible hinges (e.g., film hinges) in symmetrical positions on both sides of the needle hole 210. Due to the connection via the flexible hinges, the upper cap 22 and the lower cap 23 can move between the open sate as shown in Fig. 4 and the closed state as shown in Fig. 7. As shown in Fig. 7, a channel 24 allowing the movement of the handle portion 11 of butterfly-type needle 1 is provided after the upper cap 22 and the lower cap 23 are closed.

A clamping structure is formed between the upper cap 22 and the lower cap 23 and can match with the wing portion 13 of the butterfly-type needle 1, so as to clamp the butterfly-type needle 1 in the using state as shown in Fig. 1 or lock the same in the retracted state as shown in Fig. 8, and the clamping structure also allows the butterfly-type needle 1 to move axially to the retracted state under the action of a designed external force (applied by the wing portion 13, and if the external force is not applied, it cannot move). The clamping structure may have multiple implementations. In a preferred embodiment of the present invention, the clamping structure is as shown in Figs. 4-7, the lower edge of the upper cap 22 adjacent to the near end is formed with a guide slope 220, the guide slope 220 on one hand plays the role of preventing the accidental retraction of the butterfly-type needle 1, and on the other hand plays the role of guiding the wing portion 13 when pulling the butterfly-type needle 1 in the using state towards the near end, so that the butterfly-type needle 1 smoothly moves axially into the retracted state. The lower edge in the middle part of the upper cap 22 is provided with a downward protrusion 224, and the downward protrusion 224 protrudes until the upper edge of the lower cap 23, as shown in Fig. 7, and in this way the middle parts of the upper cap 22 and the lower cap 23 are fully enclosed structures. The lower edge of the upper cap 22 adjacent to the downward protrusion 224 is formed with a recessed portion 221 for receiving the wing portion 13, and the recessed portion 221 is provided with a side wall 2210 for matching with the wing portion 13 in a 90 degree clamped manner.

With further reference to Figs. 4-7, the lower cap 23 is formed with a first upward protrusion 231 on the inner side of the downward protrusion 224, and the first upward protrusion 231 is also used for matching with the wing portion 13 in a 90 degree clamped manner. The lower edge of the lower cap 23 below the recessed portion 221 is formed with a second upward protrusion 230, and the second upward protrusion 230 is used for clamping an orifice slot 132 of the wing portion 13.

In the retracted locked state, the wing portion 13 of the butterfly-type needle 1 match with at least one of the side wall 2210 of the recessed portion 221, the first upward protrusion 231, and the second upward protrusion 230. The second upward protrusion 230 may not be clamped into the orifice slot 132; in this case the side wall 2210 and the first upward protrusion 231 clamp the wing portion 13, and the butterfly-type needle 1 is locked in a position as shown in Fig. 8, so that the butterfly-type needle 1 cannot move forward. If the second upward protrusion 230 is clamped into the orifice slot 132, the side wall 2210, the first upward protrusion 231 and the second upward protrusion 230 play the role of clamping at the same time. The side wall 2210, the first upward protrusion 231 and the second upward protrusion 230 provide triple clamping effects, and in practice, this would completely prevent the butterfly needle 1 from releasing.

An anchor-shaped latch 223 protrudes from the near end of the upper cap 22; accordingly, the near end of the lower cap is provided with a stepped lock hole 234; the latch 223 is formed of a resilient material; and the size of the latch 223 is slightly larger than that of the lock hole 234, such that the latch 223 can be detachably clamped with the lock hole 234.

The wing portion 13 is made of a soft material; when a health care worker folds and grips the wing portion 13 during using, the wing portion 13 is softer and is easier to fold, and therefore more gripping space is obtained. When the wing portion 13 passes across the enclosed structure in the middle parts of the upper cap 22 and the lower cap 23, it would render apart the middle parts of the upper cap 22 and the lower cap 23, and thereafter the upper cap 22 and the lower cap 23 will be elastically restored and re-form the enclosed structure.

In other embodiments of the present invention, the wing portion 13 and the handle portion 11 may be integrally moulded or separately moulded and bonded by glue. The needle tip protective sleeve 21, the upper cap 22 and the lower cap 23 of the protective sleeve 2 may also be separately moulded and connected together after being folded, latched, bonded or welded.

As shown in Figs. 11 and 1, the front face 131 of the wing portion 13 and the lower side of the upper cap 22 are opposite to each other, the back face 130 and the upper side of the lower cap 23 are opposite to each other, a blade face groove of the needle tip 120 of the needle portion 12 and the front face 131 are oriented in the same direction, and the needle handle 11 is located on the back face 130 side of the wing portion 13.

As shown in Fig. 15, the handle portion 11 can be connected together with a blood collector or an infusion device once the tail end thereof is connected to the hose 3.

In the preceding embodiments of the present invention, since the protective sleeve 2 has a needle tip protective head 21, after the needle tip retracts, the periphery thereof is completely enclosed, and therefore it certainly will not cause accidental puncture injury, or the risk of infection caused by puncture. Moreover, since the protective head 21 is an integrally moulded part, the structure is simple, and the surface is rounded or smooth with no projection and having a better feel, so that better protection is provided for the user.

When the protective sleeve 2 is integrally injection-moulded, the components thereof are fewer in number, so the structure is simple, easier for mass production, and lower in costs.

The present invention has been disclosed above in terms of the preferred embodiments which, however, are not intended to limit the present invention; any person skilled in the art could make possible changes and alterations without departing from the spirit and scope of the present invention, and the aforementioned content is not limited to the venous blood collection or puncture infusion but may also be used in other medical applications. Hence, any alteration, equivalent change and modification which are made to the above-mentioned embodiments in accordance with the technical substance of the present invention and without departing from the contents of the present invention, will fall within the scope of protection defined by the claims of the present invention.

## Claims

1. A butterfly-type needle protective sleeve (2), comprising a body for placing a butterfly-type needle (1) therein, said body being made of a plastic material and integrally injection-moulded, said butterfly-type needle (1) comprising a needle portion (12), a wing portion (13) protruding out of said body, and a handle portion (11) fixed onto said wing portion (13); one end of said handle portion (11) being connected to said needle portion (12) and the other end being used for connecting to a pipe (3), and said butterfly-type needle (1), with respect to said protective sleeve (2), having a using state in which said needle portion (12) protrudes and a retracted state in which said needle portion (12) retracts,
**characterized in that**
said body comprises a needle tip protective head (21), an upper cap (22) and a lower cap; said needle tip protective head (21) is a cylinder with an axially penetrating needle hole (210); said upper cap (22) and said lower cap (23) are elongated components extending in an axial direction of said body and respectively have a far end and a near end; said far ends of said upper cap (22) and said lower cap (23) are connected to said cylinder through flexible hinges; said near end of said upper cap (22) is detachably connected with said near end of said lower cap (23) in a clamping manner; said upper cap (22) and said lower cap (23) can be allowed to move between an open state and a closed state by aid of said flexible hinges; a channel (24) for allowing said handle portion (11) to move axially is arranged between said upper cap (22) and said lower cap (23) in the closed state; a clamping structure is formed between said upper cap (22) and said lower cap (23) and can match with said wing portion (13) of said butterfly-type needle (1) to enable said butterfly-type needle (1) to be clamped in the using state or be locked in the retracted state; said butterfly-type needle (1) can be allowed to move axially to the retracted state under an outer force; said needle hole (210) of said needle tip protective head (21) is used for accommodating axial movement of said needle portion (12); a needle tip of said needle portion (12) in the retracted state is accommodated in the needle tip protective head (21), and a circumferential side corresponding to the needle tip is of a fully enclosed structure; a lower edge of said upper cap (22) adjacent to said far end is formed with a continuous guide slope (220), so as to guide said wing portion (13) when pulling said butterfly-type needle (1) in the using state towards the near end, so that said butterfly-type needle (1) smoothly moves axially into said retracted state.

2. The butterfly-type needle protective sleeve of claim 1, **characterized in that** an upper edge of said lower cap (23) adjacent to said far end is formed as a plane (232).

3. The butterfly-type needle protective sleeve (2) of claims 1 and 2, **characterized in that** the lower edge in the middle part of said upper cap (22) protrudes downward until the upper edge of said lower cap (23), such that in said closed state, the middle parts of said upper cap (22) and said lower cap (23) are of fully enclosed structures.

4. The butterfly-type needle protective sleeve (2) of claim 3, **characterized in that** the lower edge of said upper cap (22) adjacent to said downward protrusion (224) is formed with a recessed portion (221) for receiving said wing portion (13), and the recessed portion (221) is provided with a side wall (2210) for matching with said wing portion (13) in a 90 degree clamped manner.

5. The butterfly-type needle protective sleeve (2) of claim 4, **characterized in that** said lower cap (23) is formed with a first upward protrusion (231) on inner side of said downward protrusion (224), and said first upward protrusion (231) is used for matching with said wing portion (13) in a 90 degree clamped manner.

6. The butterfly-type needle protective sleeve of claim 5, **characterized in that** an upper edge of said lower cap (23) below said recessed portion(221) is formed with a second upward protrusion (230), and said second upward protrusion (230) is used for clamping an orifice slot of said wing portion (13).

7. A butterfly-type needle assembly, comprising a butterfly-type needle (1) and a butterfly-type needle protective sleeve (2) as claimed in any one of claims 1-6.

8. The butterfly-type needle assembly of claim 7, **characterized in that** said wing portion (13) has a front face (131) and a back face (130), said front face (131) and lower side of said upper cap (22) are opposite to each other, said back face (130) and upper side of said lower cap (23) are opposite to each other, a blade face groove of the needle tip of said needle portion (12) and said front face (131) are oriented in the same direction, and said needle handle is located on back face (130) side of said wing portion (13).

## Patentansprüche

1. Schmetterlingsnadel-Schutzhülle (2), die einen Körper umfasst, um eine Schmetterlingsnadel (1) darin anzubringen, wobei der Körper aus einem Kunststoffmaterial hergestellt und einstückig gespritzt ist, wobei die Schmetterlingsnadel (1) einen Nadelabschnitt (12), einen aus dem Körper hervorstehenden Flügelabschnitt (13) und einen an dem Flügelabschnitt (13) befestigten Griffabschnitt (11) umfasst; wobei ein Ende des Griffabschnitts (11) mit dem Nadelabschnitt (12) verbunden ist, und das andere Ende zum Verbinden mit einem Rohr (3) verwendet wird, und die Schmetterlingsnadel (1) in Bezug auf die Schutzhülle (2) einen Verwendungszustand aufweist, in dem der Nadelabschnitt (12) vorsteht, und einen zurückgezogenen Zustand, in dem der Nadelabschnitt (12) zurückgezogen wird,
**dadurch gekennzeichnet, dass**
der Körper einen Nadelspitzen-Schutzkopf (21), eine obere Abdeckung (22) und eine untere Abdeckung umfasst; der Nadelspitzen-Schutzkopf (21) ein Zylinder mit einem axial durchgängigen Nadelloch (210) ist; die obere Abdeckung (22) und die untere Abdeckung (23) längliche Komponenten sind, die sich in einer axialen Richtung des Körpers erstrecken und jeweils ein fernes Ende und ein nahes Ende aufweisen; die fernen Enden der oberen Abdeckung (22) und der unteren Abdeckung (23) durch flexible Scharniere mit dem Zylinder verbunden sind; das nahe Ende der oberen Abdeckung (22) lösbar mit dem nahen Ende der unteren Abdeckung (23) klemmend verbunden ist; die obere Abdeckung (22) und die untere Abdeckung (23) sich mit Hilfe der flexiblen Scharniere zwischen einem offenen Zustand und einem geschlossenen Zustand bewegen können; ein Kanal (24) zwischen der oberen Abdeckung (22) und der unteren Abdeckung (23) angeordnet ist, um dem Griffabschnitt (11) zu ermöglichen, sich im geschlossenen Zustand axial zu bewegen; eine Klemmstruktur zwischen der oberen Abdeckung (22) und der unteren Abdeckung (23) ausgebildet ist und mit dem Flügelabschnitt (13) der Schmetterlingsnadel (1) angepasst werden kann, um ein Klemmen der Schmetterlingsnadel (1) in dem Verwendungszustand oder ein Verriegeln im zurückgezogenen Zustand zu ermöglichen; die Schmetterlingsnadel (1) sich aufgrund einer äußeren Kraft axial in den zurückgezogenen Zustand bewegen kann; das Nadelloch (210) des Nadelspitzen-Schutzkopfes (21) zur Aufnahme einer axialen Bewegung des Nadelabschnitts (12) verwendet wird; eine Nadelspitze des Nadelabschnitts (12) im zurückgezogenen Zustand in dem Nadelspitzen-Schutzkopf (21) untergebracht ist, und eine Umfangsseite, die der Nadelspitze entspricht, eine vollständig geschlossene Struktur aufweist; eine untere Kante der oberen Abdeckung (22), die an das ferne Ende angrenzt, mit einer kontinuierlichen Führungsschräge (220) ausgebildet ist, um den Flügelabschnitt (13) zu führen, wenn die Schmetterlingsnadel (1) in dem Verwendungszustand gegen das nahe Ende gezogen wird, so dass sich die Schmetterlingsnadel (1) reibungslos axial in den zurückgezogenen Zustand bewegt.

2. Schmetterlingsnadel-Schutzhülle nach Anspruch 1, **dadurch gekennzeichnet, dass** eine obere Kante der unteren Abdeckung (23), die an das ferne Ende angrenzt, als eine Ebene (232) ausgebildet ist.

3. Schmetterlingsnadel-Schutzhülle (2) nach den Ansprüchen 1 und 2, **dadurch gekennzeichnet, dass** die untere Kante in dem mittleren Teil der oberen Abdeckung (22) bis zur oberen Kante der unteren Abdeckung (23) nach unten derart vorsteht, dass in dem geschlossenen Zustand die mittleren Teile der oberen Abdeckung (22) und der unteren Abdeckung (23) vollständig geschlossene Strukturen sind.

4. Schmetterlingsnadel-Schutzhülle (2) nach Anspruch 3, **dadurch gekennzeichnet, dass** die untere Kante der oberen Abdeckung (22), die an den nach unten gerichteten Vorsprung (224) angrenzt, mit einem ausgesparten Abschnitt (221) zum Aufnehmen des Flügelabschnitts (13) ausgebildet ist, und der ausgesparte Abschnitt (221) mit einer Seitenwand (2210) zum Anpassen an den Flügelabschnitt (13) in einer 90-Grad-Klemmung versehen ist.

5. Schmetterlingsnadel-Schutzhülle (2) nach Anspruch 4, **dadurch gekennzeichnet, dass** die untere Abdeckung (23) mit einem ersten nach oben gerichteten Vorsprung (231) an der Innenseite des nach unten gerichteten Vorsprungs (224) ausgebildet ist und der erste nach oben gerichtete Vorsprung (231) zum Anpassen an den Flügelabschnitt (13) in einer 90-Grad-Klemmung verwendet wird.

6. Schmetterlingsnadel-Schutzhülle nach Anspruch 5, **dadurch gekennzeichnet, dass** eine obere Kante der unteren Abdeckung (23) unter dem ausgesparten Abschnitt (221) mit einem zweiten nach oben gerichteten Vorsprung (230) ausgebildet ist und der zweite nach oben gerichtete Vorsprung (230) zum Festklemmen eines Öffnungsschlitzes des Flügelabschnitts verwendet wird (13) .

7. Schmetterlingsnadelanordnung umfassend eine Schmetterlingsnadel (1) und eine Schmetterlingsnadel-Schutzhülle (2) nach einem der Ansprüche 1-6.

8. Schmetterlingsnadelanordnung nach Anspruch 7, **dadurch gekennzeichnet, dass** der Flügelabschnitt (13) eine Vorderseite (131) und eine Rückseite (130) aufweist, wobei die Vorderseite (131) und die Unterseite der oberen Abdeckung (22) einander gegenüberliegen, wobei die Rückseite (130) und die Oberseite der unteren Abdeckung (23) einander gegenüberliegen, eine Klingenflächennut der Nadelspitze des Nadelabschnitts (12) und die Vorderseite (131) in die gleiche Richtung ausgerichtet sind, und der Nadelgriff sich auf der Rückseite (130) des Flügelabschnitts (13) befindet.

## Revendications

1. Manchon protecteur d'aiguille de type papillon (2) comprenant un corps pour placer une aiguille de type papillon (1) à l'intérieur, ledit corps étant fait d'un matériau plastique et intégralement moulé par injection, ladite aiguille de type papillon (1) comprenant une partie d'aiguille (12), une partie d'ailette (13) faisant saillie hors dudit corps, et une partie de manche (11) fixée sur ladite partie d'ailette (13) ; une extrémité de ladite partie de manche (11) étant connectée à ladite partie d'aiguille (12) et l'autre extrémité étant utilisée pour la connexion à un tuyau (3), et ladite aiguille de type papillon (1), par rapport audit manchon protecteur (2), ayant un état d'utilisation dans lequel ladite partie d'aiguille (12) fait saillie et un état rétracté dans lequel ladite partie d'aiguille (12) se rétracte,
**caractérisé en ce que**
ledit corps comprend une tête protectrice d'embout d'aiguille (21), un bouchon supérieur (22) et un bouchon inférieur ; ladite tête protectrice d'embout d'aiguille (21) est un cylindre avec un trou d'aiguille à pénétration axiale (210) ; ledit bouchon supérieur (22) et ledit bouchon inférieur (23) sont des composants allongés s'étendant dans une direction axiale dudit corps et ont respectivement une extrémité éloignée et une extrémité proche ; lesdites extrémités éloignées dudit bouchon supérieur (22) et dudit bouchon inférieur (23) sont connectées audit cylindre par des charnières flexibles ; ladite extrémité proche dudit bouchon supérieur (22) est connectée de manière séparable à ladite extrémité proche dudit bouchon inférieur (23) dans une manière de pincement ; ledit bouchon supérieur (22) et ledit bouchon inférieur (23) peuvent se déplacer entre un état ouvert et un état fermé à l'aide desdites charnières flexibles ; un canal (24) pour permettre à ladite partie de manche (11) de se déplacer axialement est agencé entre ledit bouchon supérieur (22) et ledit bouchon inférieur (23) dans l'état fermé ; une structure de pincement est formée entre ledit bouchon supérieur (22) et ledit bouchon inférieur (23) et peut correspondre à ladite partie d'ailette (13) de ladite aiguille de type papillon (1) pour permettre à ladite aiguille de type papillon (1) d'être pincée dans l'état d'utilisation ou d'être verrouillée dans l'état rétracté ; ladite aiguille de type papillon (1) peut se déplacer axialement vers l'état rétracté sous une force extérieure ; ledit trou d'aiguille (210) de ladite tête protectrice d'embout d'aiguille (21) est utilisé pour recevoir le mouvement axial de ladite partie d'aiguille (12) ; un embout d'aiguille de ladite partie d'aiguille (12) dans l'état rétracté est reçu dans la tête protectrice d'embout d'aiguille (21), et un côté circonférentiel correspondant à l'embout d'aiguille est d'une structure entièrement ceinte ; un bord inférieur dudit bouchon supérieur (22) adjacent à ladite extrémité éloignée est formé avec une pente-guide continue (220) de façon à guider ladite partie d'ailette (13) lorsque l'aiguille de type papillon (1) est tirée dans l'état d'utilisation en direction de l'extrémité arrière de façon à ce que ladite aiguille de type papillon (1) se déplace axialement en douceur jusque dans ledit état rétracté.

2. Manchon protecteur d'aiguille de type papillon selon la revendication 1, **caractérisé en ce qu'**un bord supérieur dudit bouchon inférieur (23) adjacent à ladite extrémité éloignée est formé en tant qu'un plan (232) .

3. Manchon protecteur d'aiguille de type papillon (2) selon les revendications 1 et 2, **caractérisé en ce que** le bord inférieur dans la partie centrale dudit bouchon supérieur (22) fait saillie vers le bas jusqu'au bord supérieur dudit bouchon inférieur (23), de telle sorte que dans ledit état fermé, les parties centrales dudit bouchon supérieur (22) et dudit bouchon inférieur (23) sont des structures totalement ceintes.

4. Manchon protecteur d'aiguille de type papillon (2) selon la revendication 3, **caractérisé en ce que** le bord inférieur dudit bouchon supérieur (22) adjacent à ladite saillie vers le bas (224) est formé avec une partie en creux (221) pour recevoir ladite partie d'ailette (13), et la partie en creux (221) est dotée d'une paroi latérale (2210) pour correspondre à ladite partie d'ailette (13) dans une manière pincée à 90 degrés.

5. Manchon protecteur d'aiguille de type papillon (2) selon la revendication 4, **caractérisé en ce que** ledit bouchon inférieur (23) est formé avec une première saillie vers le haut (231) sur le côté intérieur de ladite saillie vers le bas (224), et ladite première saillie vers le haut (231) est utilisée pour correspondre à ladite partie d'ailette (13) dans une manière pincée à 90 degrés.

6. Manchon protecteur d'aiguille de type papillon selon la revendication 5, **caractérisé en ce qu'**un bord supérieur dudit bouchon inférieur (23) en-dessous de ladite partie en creux (221) est formé avec une seconde saillie vers le haut (230) et ladite seconde saillie vers le haut (230) est utilisée pour serrer une fente d'orifice de ladite partie d'ailette (13).

7. Ensemble d'aiguille de type papillon, comprenant une aiguille de type papillon (1) et un manchon protecteur d'aiguille de type papillon (2) selon l'une quelconque des revendications 1-6.

8. Ensemble d'aiguille de type papillon selon la revendication 7, **caractérisé en ce que** ladite partie d'ailette (13) a une face avant (131) et une face arrière (130), lesdits face avant (131) et côté inférieur dudit bouchon supérieur (22) sont opposés l'un à l'autre, lesdits face arrière (130) et côté supérieur dudit bouchon inférieur (23) sont opposés l'un à l'autre, une rainure de face de lame dudit embout d'aiguille de ladite partie d'aiguille (12) et ladite face avant (131) sont orientés dans la même direction, et ledit manche d'aiguille est situé sur le côté face arrière (130) de ladite partie d'ailette (13) .
